# EUROPEAN PATENT SPECIFICATION

(11) **EP 2 675 520 B1**
(45) Date of publication and mention of the grant of the patent: **17.12.2014**
(21) Application number: 12706375.8
(22) Date of filing: 13.02.2012
(51) Int. Cl.: A61N 1/05, A61B 17/34, A61M 25/06, A61M 29/00, A61M 25/01

(54) **SYSTEMS AND METHODS FOR IMPLANTING PADDLE LEAD ASSEMBLIES OF ELECTRICAL STIMULATION SYSTEMS**
SYSTEME UND VERFAHREN ZUR IMPLANTATION VON PADDELELEKTRODENANORDNUNGEN ELEKTRISCHER STIMULATIONSSYSTEME
SYSTÈMES ET PROCÉDÉS PERMETTANT L'IMPLANTATION D'ENSEMBLES DE DÉRIVATION À PALETTE DE SYSTÈMES DE STIMULATION ÉLECTRIQUE

(30) Priority: 16.02.2011 US 201161443358 P
(43) Date of publication of application: 25.12.2013
(73) Proprietor: Boston Scientific Neuromodulation Corporation, Valencia, CA 91355 (US)
(72) Inventor: BARKER, John, Michael, Ventura, CA 93003 (US); HOWARD, Joshua, Dale, Granada Hills, California 91344 (US)
(74) Representative: Vossius & Partner
(86) International application number: PCT/US2012/024835
(87) International publication number: WO 2012/112428

(56) References cited:
- EP-A1- 1 048 270
- WO-A1-2005/023359
- WO-A2-2006/119135
- US-A- 4 512 351
- US-A1- 2011 224 680

## Description

### CROSS-REFERENCE TO RELATED APPLICATIONS

This application claims the benefit of U.S. Provisional Patent Application Serial No. 6.1/443,358 filed on February 16, 2011.

### FIELD

The present invention is directed to the area of implantable electrical stimulation systems and methods of making and using the systems, The present invention is also directed to lead introducers for facilitating percutaneous implantation of implantable electrical stimulation paddle leads, as well as methods of making and using the introducers, paddle leads, and electrical stimulation systems.

### BACKGROUND

Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat incontinence, as well as a number of other applications under investigation. Functional electrical stimulation systems have been applied to restore some functionality to paralyzed extremities in spinal cord injury patients.

Stimulators have been developed to provide therapy for a variety of treatments. A stimulator can include a control module (with a pulse generator), one or more leads, and an array of stimulator electrodes on each lead. The stimulator electrodes are in contact with or near the nerves, muscles, or other tissue to be stimulated. The pulse generator in the control module generates electrical pulses that are delivered by the electrodes to body tissue.

US 4,512,351 A discloses a method and apparatus for introducing a neural stimulator into the epidural space in the spinal cord of a patient. The apparatus includes an introducer into which an epidural needle assembly may be inserted through a cannula. The combination of the introducer and the epidural needle assembly is introduced in between the vertebrae of the spinal cord. The epidural needle assembly includes a needle which in turn has a cannula and an obturator. The needle is inserted along with the epidural needle assembly between the vertebrae. The obturator is removed from the cannula of the needle, and the cannula is filled with saline solution. Thereafter, the introducer is advanced in the epidural space while the needle is withdrawn from the cannula. The neural stimulator is inserted through the cannula of the introducer and positioned appropriately at the epidural space. The introducer is then split away from the electrode by a pair of handles.

WO 2006/119135 A2 discloses an introducer that is provided for implanting a paddle style electrical stimulation lead for a nerve tissue. The introducer includes an outer sheath and inner penetrator. The inner penetrator includes an inner channel accommodating a guide wire, a tip end, a body region and one or more transition regions connecting the tip end and the body region. The inner penetrator may be advanced along the guide wire to a desired location in relation to the nerve tissue. Thereafter, the inner penetrator is removed from the outer sheath leaving the outer sheath substantially in position for insertion of the stimulation lead in proximity to the nerve tissue.

### BRIEF SUMMARY

In one embodiment, an insertion kit for percutaneously implanting an electrical stimulating paddle lead into a patient includes a paddle lead introducer. The paddle lead introducer is configured and arranged for facilitating percutaneous implantation of a paddle lead into the patient. The paddle lead introducer includes a sheath and a dilator. The sheath is insertable into the patient. The sheath has a first end, an opposing second end, and a longitudinal length. The sheath is configured and arranged to receive a paddle lead during implantation of the paddle lead into the patient. The sheath is configured and arranged to divide into at least two parts for removal of the sheath from the paddle lead upon implantation of the paddle lead into the patient. The dilator is insertable into the sheath. The dilator has a first end, an opposing second end, and a longitudinal length. The first end of the dilator defines an aperture at a tip of the first end. The first end of the dilator has a transverse circumference that increases from the tip towards the second end.

A method for percutaneously implanting an electrical stimulation paddle lead into a patient includes providing an insertion kit is disclosed. The insertion kit includes a paddle lead introducer. The paddle lead: introducer is configured and arranged for facilitating percutaneous implantation of a paddle lead into the patient. The paddle lead introducer includes a sheath and a dilator. The sheath is insertable into the patient. The sheath has a first end, an opposing second end, and a longitudinal length. The sheath is configured and arranged to receive a paddle lead during implantation of the paddle lead into the patient. The sheath is configured and arranged to divide into at least two parts for removal of the sheath from the paddle lead upon implantation of the paddle lead into the patient. The dilator is insertable into the sheath. The dilator has a first end, an opposing second end, and a longitudinal length. The first end of the dilator defines an aperture at a tip of the first end. The first end of the dilator has a transverse circumference that increases from the tip towards the second end. The dilator of the insertion kit is inserted into the sheath such that the first end of the dilator extends axially from the first end of the sheath. An epidural needle is inserted into the dilator such that a first end of the epidural needle extends axially from the first end of the dilator. The sheath, dilator, and epidural needle are guided to a target stimulation location within the patient. The epidural needle and dilator are removed, leaving the sheath in the patient. The paddle lead is inserted into the sheath and guided to the target stimulation location. The paddle lead includes a paddle body having a first major surface, an opposing second major surface, a longitudinal length, and at least one lead body. Each of the at least one lead bodies has a proximal end and a distal end. The distal end of the at least one lead body is coupled to the paddle body. A plurality of electrodes are disposed on the first major surface of the paddle body. A plurality of terminals are disposed at the proximal ends of each of the at least one lead bodies. A plurality of conductive wires couple the plurality of electrodes electrically to the plurality of terminals. The sheath is removed from the paddle lead, leaving the paddle lead implanted in the patient.

A method for percutaneously implanting an electrical stimulation paddle lead into a patient includes providing an insertion kit. The insertion kit includes a paddle lead introducer. The paddle lead introducer is configured and arranged for facilitating percutaneous implantation of a paddle lead into the patient. The paddle lead introducer includes a sheath and a dilator. The sheath is insertable into the patient. The sheath has a first end, an opposing second end, and a longitudinal length. The sheath is configured and arranged to receive a paddle lead during implantation of the paddle lead into the patient. The sheath is configured and arranged to divide into at least two parts for removal of the sheath from the paddle lead upon implantation of the paddle lead into the patient. The dilator is insertable into the sheath. The dilator has a first end, an opposing second end, and a longitudinal length. The first end of the dilator defines an aperture at a tip of the first end. The first end of the dilator has a transverse circumference that increases from the tip towards the second end. The dilator of the insertion kit is inserted into the sheath such that the first end of the dilator extends axially from the first end of the sheath. An epidural needle is inserted into the patient and guided to the target stimulation location. A lead blank is inserted into the epidural needle. The epidural needle is removed, leaving the lead blank in the patient. The sheath and the dilator of the insertion kit are disposed over the lead blank. The dilator and lead blank are removed, leaving the sheath in the patient. The paddle lead is inserted into the sheath and guided to the target stimulation location. The paddle lead includes a paddle body having a first major surface, an opposing second major surface, a longitudinal length, and at least one lead body. Each of the at least one lead bodies has a proximal end and a distal end. The distal end of the at least one lead body is coupled to the paddle body. A plurality of electrodes are disposed on the first major surface of the paddle body. A plurality of terminals are disposed at the proximal ends of each of the at least one lead bodies. A plurality of conductive wires couple the plurality of electrodes electrically to the plurality of terminals. The sheath is removed from the paddle lead, leaving the paddle lead implanted in the patient.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed. Description, which is to be read in association with the accompanying drawings, wherein:
FIG. 1 is a schematic view of one embodiment of an electrical stimulation system that includes a paddle body coupled to a control module via lead bodies, according to the invention;
FIG. 2A is a schematic side view of one embodiment of a plurality of connector assemblies disposed in the control module of FIG. 1, the connector assemblies configured and arranged to receive the proximal portions of the lead bodies of FIG. 1, according to the invention;
FIG. 2B is a schematic side view of one embodiment of a proximal portion of a lead body and a lead extension coupled to a control module, the lead extension configured and arranged to couple the proximal portion of the lead body to the control module, according to the invention;
FIG. 2C is a schematic side view of one embodiment of a connector assembly disposed in the control module of FIG. 2B, the connector assembly configured and arranged to receive the lead extension of FIG. 2B, according to the invention;
FIG. 3 is a schematic longitudinal cross-sectional view of one embodiment of one of the connector assemblies of FIG. 1, according to the invention.
FIG. 4 is a schematic perspective view a control module with a header that defines four ports, according to the invention;
FIG. 5A is a schematic perspective view of one embodiment of a lead introducer suitable for percutaneous implantation of a paddle lead into a patient, the introducer including a sheath and a dilator, according to the invention;
FIG. 5B is a schematic perspective view of one embodiment of a lead blank inserted into an epidural needle which, in turn, is inserted into the lead introducer of FIG. 5A, according to the invention;
FIG. 6 is a schematic top view of one embodiment of the paddle body of FIG. 1 and the lead blank of FIG. 5B, the paddle body defining an aperture configured and arranged to receive the lead blank, according to the invention;
FIG. 7 is a schematic perspective view of one embodiment of the paddle body of FIG. 1 partially disposed in the sheath of FIG. 5A, according to the invention;
FIG. 8 is a schematic perspective view of one embodiment of the sheath of FIG. 5A being removed from the paddle body of FIG. 1, according to the invention; and
FIG. 9 is a schematic overview of one embodiment of components of a stimulation system, including an electronic subassembly disposed within a control module, according to the invention.

### DETAILED DESCRIPTION

The present invention is directed to the area of implantable electrical stimulation systems and methods of making and using the systems. The present invention is also directed to lead introducers for facilitating percutaneous implantation of implantable electrical stimulation paddle leads, as well as methods of making and using the introducers, paddle leads, and electrical stimulation systems.

Suitable implantable electrical stimulation systems include, but are not limited to, an electrode lead ("lead") with one or more electrodes disposed on a distal end of the lead and one or more terminals disposed on one or more proximal ends of the lead. Leads include, for example, percutaneous leads, paddle leads, and cuff leads. Examples of electrical stimulation systems with leads are found in, for example, U.S. Patents Nos. 6,181,969; 6,516,227; 6,609,029; 6,609,032; and 6,741,892; 7,244,150; 7,672,734; 7,761,165; 7,949,395; and 7,974,706; and U.S. Patent Applications Publication Nos. 2005/0165465, 2007/0150036; 2007/0219595; and 2008/0071320.

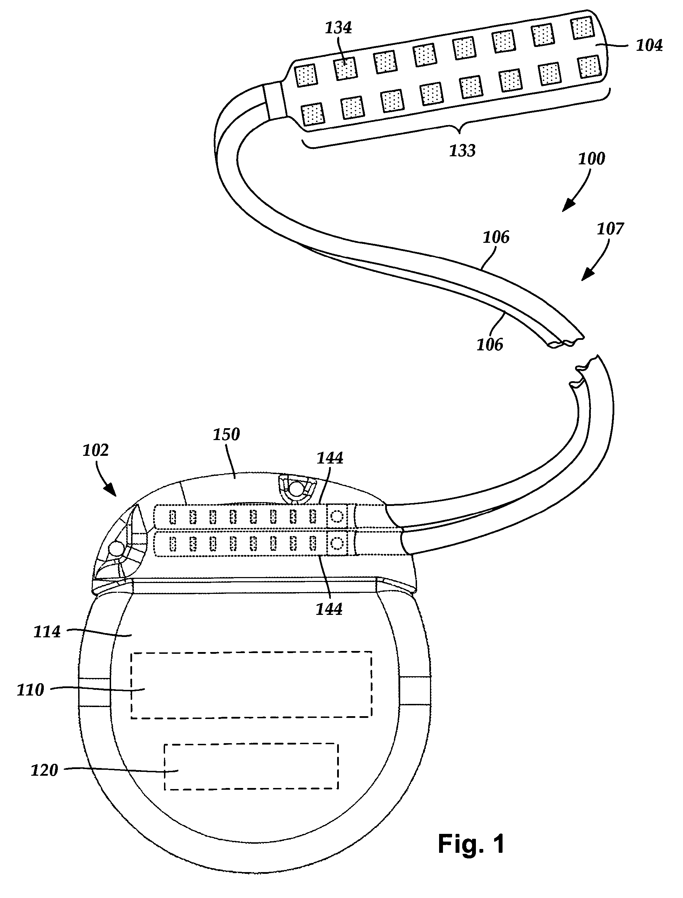

Figure 1 illustrates schematically one embodiment of an electrical stimulation system 100. The electrical stimulation system includes a control module (*e.g.*, a stimulator or pulse generator) 102, a paddle body 104, and one or more lead bodies 106 coupling the control module 102 to the paddle body 104. The paddle body 104 and the one or more lead bodies 106 collectively form a paddle lead 107. The paddle body 104 typically includes a plurality of electrodes 134 that form an array of electrodes 133. The control module 102 typically includes an electronic subassembly 110 and an optional power source 120 disposed in a sealed housing 114. In Figure 1, two lead bodies 106 are shown coupled to the control module 102.

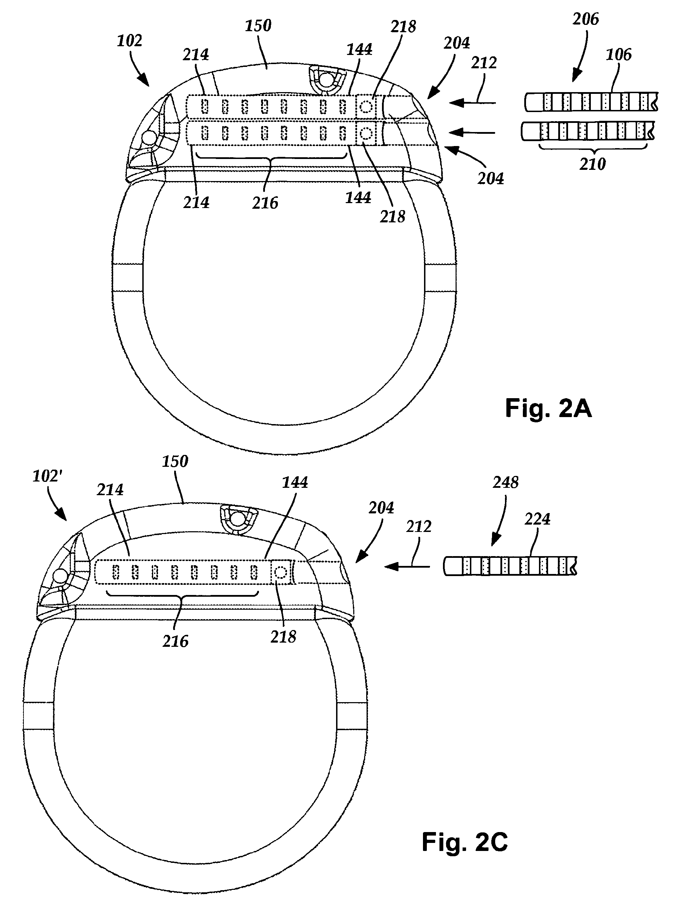

The control module 102 typically includes one or more connector assemblies 144 into which the proximal end of the one or more lead bodies 106 can be plugged to make an electrical connection via connector contacts (*e.g.*, 216 in Figure 2A). The connector contacts are coupled to the electronic subassembly 110 and the terminals are coupled to the electrodes 134. In Figure 1, two connector assemblies 144 are shown.

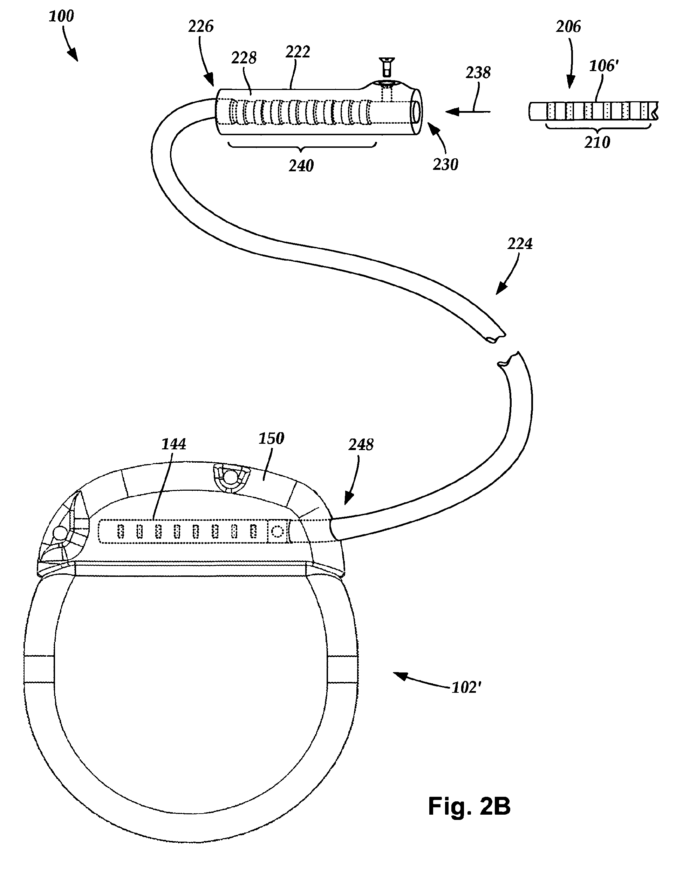

The one or more connector assemblies 144 may be disposed in a header 150. The header 150 provides a protective covering over the one or more connector assemblies 144. The header 150 may be formed using any suitable process including, for example, casting, molding (including injection molding), and the like. In addition, one or more lead extensions 224 (see Figure 2B) can be disposed between the one or more lead bodies 106 and the control module 102 to extend the distance between the one or more lead bodies 106 and the control module 102.

The electrical stimulation system or components of the electrical stimulation system, including one or more of the lead bodies 106, the paddle body 104, and the control module 102, are typically implanted into the body of a patient. The electrical stimulation system can be used for a variety of applications including, but not limited to, spinal cord stimulation, brain stimulation, neural stimulation, muscle activation via stimulation of nerves innervating muscle, and the like.

The electrodes 134 can be formed using any conductive, biocompatible material. Examples of suitable materials include metals, alloys, conductive polymers, conductive carbon, and the like, as well as combinations thereof. In at least some embodiments, one or more of the electrodes 134 are formed from one or more of: platinum, platinum iridium, palladium, titanium nitride, or rhenium.

The number of electrodes 134 in the array of electrodes 133 may vary. For example, there can be two, three, four, five, six, seven, eight, nine, ten, eleven, twelve, thirteen, fourteen, fifteen, sixteen, or more electrodes 134. As will be recognized, other numbers of electrodes 134 may also be used. In Figure 1, sixteen electrodes 134 are shown. The electrodes 134 can be formed in any suitable shape including, for example, round, oval, triangular, rectangular, pentagonal, hexagonal, heptagonal, octagonal, or the like.

The electrodes of the paddle body 104 or one or more lead bodies 106 are typically disposed in, or separated by, a non-conductive, biocompatible material including, for example, silicone, polyurethane, and the like or combinations thereof. The paddle body 104 and one or more lead bodies 106 may be formed in the desired shape by any process including, for example, molding (including injection molding), casting, and the like. Electrodes and connecting wires can be disposed onto or within a paddle body either prior to or subsequent to a molding or casting process. The non-conductive material typically extends from the distal end of the lead body to the proximal end of each of the one or more lead bodies 106. The non-conductive, biocompatible material of the paddle body 104 and the one or more lead bodies 106 may be the same or different. The paddle body 104 and the one or more lead bodies 106 may be a unitary structure or can be formed as two separate structures that are permanently or detachably coupled together.

Terminals (*e.g.*, 210 in Figure 2A) are typically disposed at the proximal end of the one or more lead bodies 106 for connection to corresponding conductive contacts (*e.g.*, 216 in Figure 2A) in connector assemblies disposed on, for example, the control module 102 (or to other devices, such as conductive contacts on a lead extension, an operating room cable, a lead splitter, a lead adaptor, or the like). Conductive wires (not shown) extend from the terminals to the electrodes 134. Typically, one or more electrodes 134 are electrically coupled to a terminal (*e.g.*, 210 in Figure 2A). In some embodiments, each terminal (*e.g.*, 210 in Figure 2A) is only coupled to one electrode 134.

The conductive wires may be embedded in the non-conductive material of the paddle lead or can be disposed in one or more lumens (not shown) extending along the paddle lead. In some embodiments, there is an individual lumen for each conductive wire. In other embodiments, two or more conductive wires may extend through a lumen. There may also be one or more lumens (not shown) that open at, or near, the proximal end of the paddle lead, for example, for inserting a stylet rod to facilitate placement of the paddle lead within a body of a patient. Additionally, there may also be one or more lumens (not shown) that open at, or near, the distal end of the paddle lead, for example, for infusion of drugs or medication into the site of implantation of the paddle body 104. The one or more lumens may, optionally, be flushed continually, or on a regular basis, with saline, epidural fluid, or the like. The one or more lumens can be permanently or removably sealable at the distal end.

As discussed above, the one or more lead bodies 106 may be coupled to the one or more connector assemblies 144 disposed on the control module 102. The control module 102 can include any suitable number of connector assemblies 144 including, for example, two three, four, five, six, seven, eight, or more connector assemblies 144. It will be understood that other numbers of connector assemblies 144 may be used instead. In Figure 1, each of the two lead bodies 106 includes eight terminals that are shown coupled with eight conductive contacts disposed in a different one of two different connector assemblies 144.

Figure 2A is a schematic side view of one embodiment of the two lead bodies 106 shown in Figure 1 configured and arranged for coupling with the control module 102. A plurality of connector assemblies 144 are disposed in the control module 102. In at least some embodiments, the control module 102 includes two, three, four, or more connector assemblies 144. Typically, the number of connector assemblies 144 disposed in the control module 102 is equal to the number of lead bodies 106 of the paddle lead. For example, in Figure 2A, the two lead bodies 106 shown in Figure 1 are shown configured and arranged for insertion into two connector assemblies 144 disposed on the control module 102.

The connector assemblies 144 each include a connector housing 214 and a plurality of connector contacts 316 disposed therein. Typically, the connector housing 214 defines a port (not shown) that provides access to the plurality of connector contacts 216. In at least some embodiments, the connector assemblies 144 further include retaining elements 218 configured and arranged to fasten the corresponding lead bodies 206 to the connector assemblies 144 when the lead bodies 106 are inserted into the connector assemblies 144 to prevent undesired detachment of the lead bodies 106 from the connector assemblies 144. For example, the retaining elements 218 may include apertures through which fasteners (*e.g.*, set screws, pins, or the like) may be inserted and secured against an inserted lead body (or lead extension).

In Figure 2A, the plurality of connector assemblies 144 are disposed in the header 150. In at least some embodiments, the header 150 defines one or more ports 204 into which a proximal end 206 of the one or more lead bodies 106 with terminals 210 can be inserted, as shown by directional arrows,212, in order to gain access to the connector contacts 216 disposed in the connector assemblies 144.

When the lead bodies 106 are inserted into the ports 204, the connector contacts 216 can be aligned with the terminals 210 disposed on the lead bodies 106 to electrically couple the control module 102 to the electrodes (134 of Figure 1) disposed at a distal end of the lead bodies 106. Examples of connector assemblies in control modules are found in, for example, U.S. Patent No. 7,244,150 and U.S. Patent Application Publication No. 2008/0071320, which are incorporated by reference.

In some instances, the electrical stimulation system may include one or more lead extensions. Figure 2B is a schematic side view of one embodiment of a proximal end of a single lead body 106' configured and arranged to couple with a lead extension 224 that is coupled with the control module 102'. In Figure 2B, a lead extension connector assembly 222 is disposed at a distal end 226 of the lead extension 224. The lead extension connector assembly 222 includes a contact housing 228. The contact housing 228 defines at least one port 230 into which a proximal end 206 of the lead body 106' with terminals 210 can be inserted, as shown by directional arrow 238. The lead extension connector assembly 222 also includes a plurality of connector contacts 240. When the lead body 106' is inserted into the port 230, the connector contacts 240 disposed in the contact housing 228 can be aligned with the terminals 210 on the lead body 106 to electrically couple the lead extension 224 to electrodes (not shown) disposed on the lead body 106'.

The proximal end of a lead extension can be similarly configured and arranged as a proximal end of a lead body, such as one of the lead bodies 106, or the lead body 106'. The lead extension 224 may include a plurality of conductive wires (not shown) that electrically couple the connector contacts 240 to terminals at the proximal end 248 of the lead extension 224. The conductive wires disposed in the lead extension 224 can be electrically coupled to a plurality of terminals (not shown) disposed on the proximal end 248 of the lead extension 224.

Figure 2C is a schematic side view of one embodiment of the lead extension 224 configured and arranged for coupling with the control module 102'. The control module 102' includes a single connector assembly 144. Alternately, the control module 102' may receive the lead body 106' directly. It will be understood that the control modules 102 and 102' can both receive either lead bodies or lead extensions. It will also be understood that the electrical stimulation system 100 can include a plurality of lead extensions 224. For example, each of the lead bodies 106 shown in Figures 1 and 2A can, alternatively, be coupled to a different lead extension 224 which, in turn, are each coupled to different ports of a two-port control module, such as the control module 102 of Figures 1 and 2A.

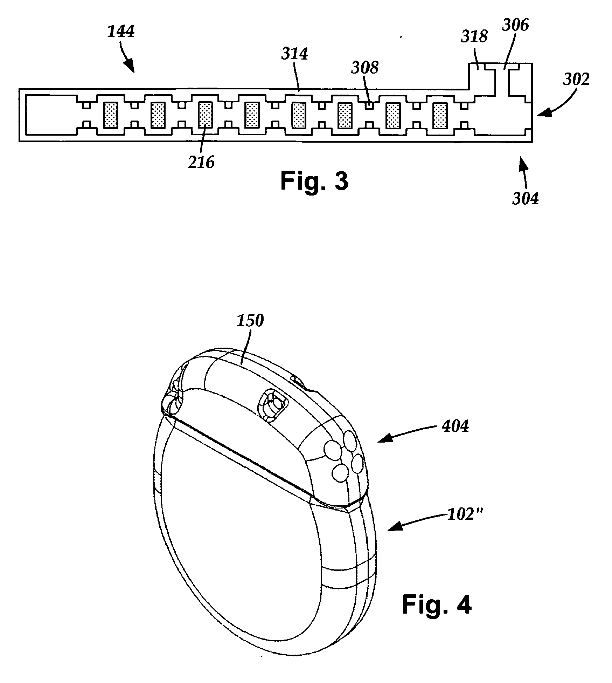

Figure 3 is a schematic longitudinal cross-sectional view of one embodiment of one of the connector assemblies 144. The connector assembly 144 includes the connector housing 314 into which a lead body or lead extension can be inserted via a port 302 at a distal end 304 of the connector housing 314. In at least some embodiments, a retaining element 318 is coupled to the connector housing 314. The retaining element 318 defines an aperture 306 through which a fastener (*e.g.*, a set screw, pin, or the like) may be inserted and secured against a lead body or lead extension when the lead body or lead extension is inserted into the port 302. Connector contacts, such as the connector contact 21.6, are disposed in the connector housing 314. In at least some embodiments, each of the connector assemblies 144 includes eight connector contacts.

The connector contacts 216 may be separated from one another by one or more non-conductive spacers (or seals), such as spacer 308, to prevent electrical contact between adjacent connector contacts 216. As discussed above, when a proximal end of a lead body or lead extension is inserted into the port 302, terminals disposed on the inserted lead body or lead extension align with the connector contacts 216, thereby establishing an electrical connection between the electronic subassembly 110 of the control module 102 and the electrodes 134 of the paddle body.

Figure 4 is a schematic perspective view of a control module 102". The header 150 of the control module 102" defines four header ports 404. Collectively, the header ports 404 are configured and arranged to each receive one or more lead bodies 106 or one or more lead extensions (*e.g.*, lead extension 224 of Figure 2B), or both. The header 150 can define any suitable number of header ports 404 including, for example, one, two, three, four, five, six, seven, eight, or more header ports 404. In Figure 4, the header 150 is shown defining four header ports 404. Thus, in at least some embodiments, the control module 102" of Figure 4 is configured and arranged to receive up to four lead bodies 106 or lead extensions 224, or a combination of both.

The header ports 404 can be defined in the header 150 in any suitable arrangement. In preferred embodiments, each of the header ports 404 are configured and arranged to align with one of the ports 302 of the one or more connector assemblies 144 disposed in the header 150. For example, in at least some embodiments, four connector assemblies 144 are disposed in the header 150 such that four header ports 404 defined in the header 150 align with the four ports 302 of the four connector assemblies 144. In at least some embodiments, the number of header ports 404 is no greater than the number of connector assemblies 144. In at least some embodiments, the number of header ports 404 is no less than the number of connector assemblies 144. In at least some embodiments, the number of header ports 404 is equal to the number of connector assemblies 144.

Conventional paddle leads are typically implanted in a patient using a laminectomy. Laminectomies are invasive procedures. Additionally, laminectomies can be expensive and time-consuming. Moreover, in many regions laminectomies are only performable by neurosurgeons, thereby potentially making the procedure more difficult to schedule.

As herein described, a percutaneous paddle lead introducer ("introducer") enables percutaneous implantation of a paddle lead into a patient. It may be advantageous to be able to implant paddle leads percutaneously in lieu of performing a laminectomy: Percutaneous implantations may be less invasive. Additionally, percutaneous implantations may be less expensive and may be performed by either neurosurgeons or anesthesiologists.

The disclosed introducer includes a sheath at least partially disposed around a dilator. An epidural needle can be inserted into the dilator and used to initiate a path through patient tissue to a target implantation location (*e.g.*, within the epidural space of the patient). The dilator can then be used to push apart enough patient tissue to enable the sheath to move along the path formed by the epidural needle to the target stimulation location. Once the sheath is at the target stimulation location, the paddle lead can be inserted into the sheath, guided to the target stimulation location, and implanted.

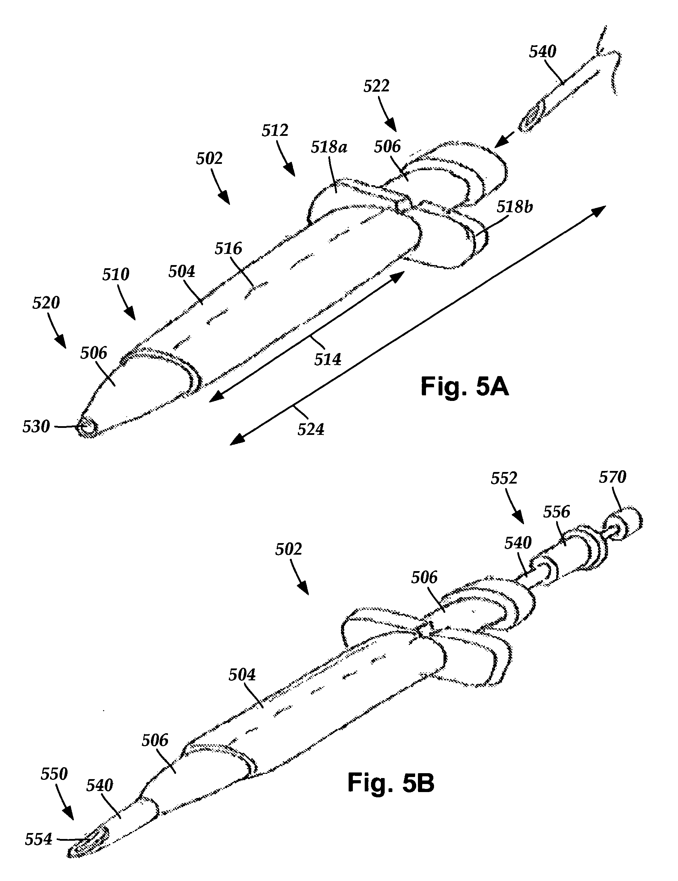

Figure 5A is a schematic perspective view of one embodiment of an introducer 502 suitable for percutaneous implantation of a paddle lead into a patient. The introducer 502 includes a sheath 504 and a dilator 506. The sheath 504 is generally cylindrical with a first end 510, an opposing second end 512, and a longitudinal length 514. The sheath 504 defines a lumen that is configured and arranged to receive the dilator 506 during insertion of the introducer 502, and that is also configured and arranged to receive the paddle body (104 in Figure 1) during implantation of the paddle body. In at least some embodiments, the sheath 504 is configured and arranged to receive the paddle body without bending the paddle body. The sheath 504 can have any suitable transverse cross-sectional shape including, for example, oval, round, rectangular, or the like. In at least some embodiments, the sheath 504 has at least one transverse axis that is no smaller than a width of the paddle lead (104 of Figure 1).

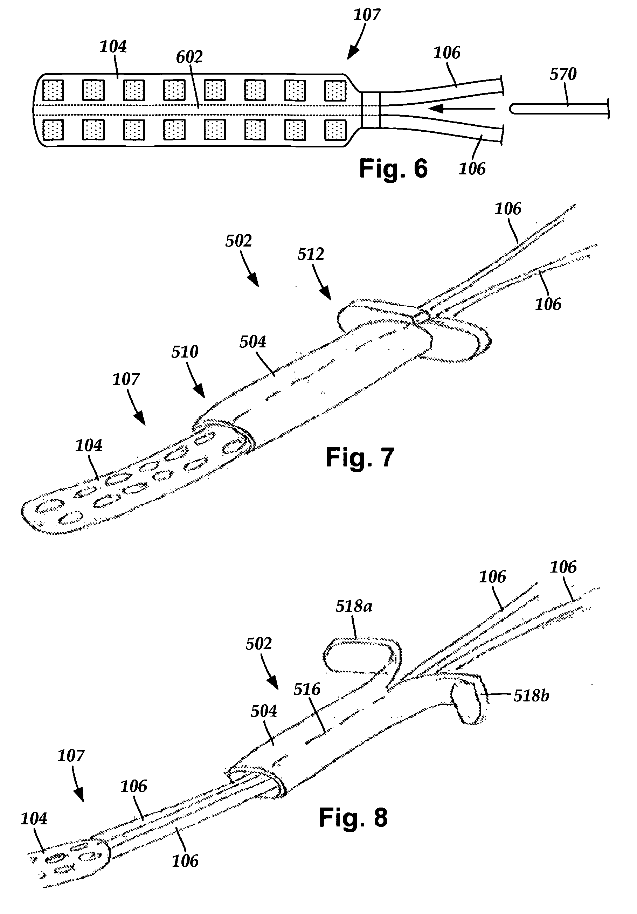

The sheath 504 can, optionally, include one or more scored or perforated lines 516 extending along the length 514 of the sheath 504. The sheath 504 can, optionally, include tabs 518a, 518b disposed at the second end 512 of the sheath 504. As. discussed in further detail below with respect to Figure 8 (and as shown in Figure 8), in at least some embodiments once the paddle lead (104 in Figure 1) is positioned at the target stimulation location, the sheath 504 can be removed from the paddle lead by separating the tabs 518a, 518b from one another, thereby causing the sheath 504 to separate along the scored or perforated lines 516 into two or more pieces that can then be removed from the patient.

The dilator 506, likewise, is generally cylindrical with a first end 520, an opposing second end 522, and a longitudinal length 524. The first end 520 of the dilator 506 defines an aperture 530 at a tip of the first end 520 and has a transverse circumference that expands axially from the tip toward the second end 522 (*i.e.*, the first end 520 is funnel-shaped). The dilator 506 can have any suitable transverse cross-sectional shape including, for example, oval, round, rectangular, or the like.

The sheath 504 is configured and arranged to mate with the dilator 506 such that the sheath 504 is disposed over at least a portion of the dilator 506. In some embodiments, the dilator 506 nests within the sheath 504. In preferred embodiments, the transverse cross-sectional shape of the dilator 506 matches the transverse cross-sectional shape of the sheath 504 to facilitate nesting of the dilator 506 within the sheath 504. The sheath 504 mates with the dilator 506 such that the first end 522 of the dilator 506 extends axially from the first end 512 of the sheath 504. The first end 510 of the sheath 504 can, optionally, be beveled or can include at least one sharp edge to facilitate the pushing apart of patient tissue during insertion of the introducer 502 into the patient.

In at least some embodiments, the dilator 506 is configured and arranged to receive an epidural needle 540. The epidural needle 540 is insertable into the second end 522 of the dilator 506 such that one end of the epidural needle 540 can be extended axially from the aperture 530. The epidural needle 540 can be of any suitable bore including, for example, 14-gauge, 15-gauge, 16-gauge, 17-gauge, 18-gauge, 19-gauge, or larger.

Figure 5B is a schematic perspective view of one embodiment of the epidural needle 540 disposed in the introducer 502. The epidural needle 540 has a first end 550 and an opposing second end 552. The epidural needle 540 defines a lumen 554 extending along a length of the epidural needle 540 between the first end 550 and the second end 552. The lumen 554 of the epidural needle 540 can, optionally, be configured and arranged to receive a guide wire or lead blank 570.

The epidural needle 540 is insertable into the dilator 506 such that the first end 550 of the epidural needle 540 is extendable through the aperture 530 at the first end 520 of the dilator 506. The first end 550 of the epidural needle 540 can be beveled to form a sharpened surface that facilitates initiating a path through patient tissue during insertion of the introducer 502 into a patient. The second end 552 of the epidural needle 540 can include a luer hub 556 configured and arranged to receive a syringe. For example, during insertion of the introducer 502, fluid (*e.g.*, saline solution, air, or the like) may be introduced or removed through the luer hub 556 to check for precise positioning of the introducer 502 (*e.g.*, in an epidural space of the patient).

The epidural needle 540 can be inserted into the dilator 506, and the introducer 502 and epidural needle 540 can be inserted into the patient in proximity to the target stimulation region. The epidural needle 540 initiates a path and the dilator 506 pushes aside enough patient tissue to enable the sheath 504 to travel down the path. Once the introducer 502 is in proximity to a target stimulation location, the positioning of the introducer 502 may be checked (*e.g.*, to confirm that the introducer 502 is disposed in an epidural space of the patient).

The positioning of the introducer 502 may be checked in any suitable manner, such as by introducing or removing fluid through the luer hub 556 (*e.g.*, performing a loss of resistance test), imaging (*e.g.*, via fluoroscopy, magnetic resonance imaging, or the like) the patient with or without introducing one or more contrast agents into the patient, or using the electrodes of the lead (or another insertable stimulation device) to stimulate surrounding patient tissue.

Optionally, at least one of the sheath 504 or the dilator 506 includes one or more radiopaque materials, for example, barium sulfate and bismuth subcarbonate, and the like or combinations thereof, that are incorporated into the introducer 502 to facilitate implantation of the paddle lead 107 through the use of one or more medical imaging techniques, such as fluoroscopy.

Once the positioning of the introducer 502.is confined, the lead blank 570 can be inserted into the epidural needle 540 and guided to the target stimulation location. Once the desired pathway is established, the dilator 506 and epidural needle 540 can be removed along the second end 512 of the sheath 504, thereby leaving the sheath 504 in proximity to the target stimulation location with the lead blank 570 disposed in the sheath 504.

The paddle lead 107 can then be inserted into the sheath 504 and guided to the target stimulation location. The lead blank 570 can be used to facilitate guidance of the paddle body 104 along the sheath 504. Figure 6 is a schematic top view of one embodiment of one end of the lead blank 570 and a distal end of the paddle lead 107. The distal end of the paddle lead 107 includes the lead bodies 106 coupled to the paddle body 104. A paddle-body aperture 602 is defined along a length of the paddle body 104. The paddle-body aperture 602 can be configured and arranged for receiving the lead blank 570. Thus, the paddle body 104 can be threaded along the lead blank 570 while the paddle lead 104 is being guided along the sheath 504 to the target stimulation location.

Optionally, one or more stylets can be used in addition to, or in lieu of, the lead blank 570 to facilitate guidance of the paddle body 104 along the sheath 504. For example, it may be useful to use one or more stylets with a paddle lead with a single tail. The one or more stylets can be inserted into one or more lumens defined in one or more of the lead bodies 106 to stiffen the one or more lead bodies 106, thereby facilitating guidance of the paddle lead 107 along the sheath 504. Alternately, the paddle body 104 can be inserted into the sheath 504 without using either the lead blank 570 or one or more stylets to facilitate guidance of the paddle body 104.

Once the paddle body 104 is inserted into the sheath 504, the lead blank 570 can be removed. Figure 7 is a schematic perspective view of one embodiment of a portion of the paddle lead 107 partially inserted into the sheath 504. In Figure 7, the paddle lead 107 is shown partially inserted into the sheath 504 such that the paddle body 104 extends from the first end 510 of the sheath 504 and the lead bodies 106 extends from the second end 512. Optionally, the one or more stylets inserted into one or more lumens of the lead bodies 106 may be used to adjust or reposition the paddle body 104 at, or around, the target stimulation location.

It will be understood that other techniques can be used for inserting the paddle lead 107 into the sheath 504. For example, in an alternate embodiment the epidural needle 540 can be inserted into the patient's epidural space without the sheath 504 or the dilator 506. Optionally, entry of the epidural needle 540 into the epidural space can be verified, as discussed above. The lead blank 570 can be inserted through the epidural needle 540. The epidural needle 540 can be removed and the sheath 504 and dilator 506 can be placed over the lead blank 570. The dilator 506 and the lead blank 570 can be removed, leaving the sheath 504 in the patient. The paddle lead 107 can then be inserted into the sheath 504.

Turning to Figure 8, once the paddle lead 104 is inserted in the sheath 504 and repositioned (if necessary), the sheath 504 can be removed from the paddle lead 107. The sheath 504 can be removed in any convenient manner. For example, the sheath 504 can be slid proximally along the paddle lead 107, or the sheath 504 can be split apart, or otherwise cut, and removed (*e.g.*, tom along the scored or perforated lines 516 or areas where it preferentially tears along a certain direction).

For example, Figure 8 is a schematic perspective view of one embodiment of the sheath 504 being removed from the paddle body 104 by separating the tabs 518a, 518b from one another. Separating the tabs 518a, 518b causes the sheath 504 to separate along the scored or perforated lines 516, thereby causing the sheath 504 to separate into two or more pieces that can be removed from the patient.

The sheath 504 can be concurrently pulled proximally along the paddle lead 107 as the tabs 518a, 518b are being separated. Eventually, the sheath 504 may be completely separated into two or more longitudinal strips, thereby separating completely from the paddle lead 107 and also from the patient. The sheath 504 can be extracted from the patient as the sheath 504 is split apart. The sheath 504 can be split apart without causing the paddle lead 107 to move. Optionally, the one or more stylets inserted into one or more lumens of the lead bodies 106 may be used to adjust the positioning of the paddle body 104 at the target stimulation location, if needed, and can then be removed from the one or more lead bodies 106.

Once the paddle lead 107 is positioned at the target stimulation site and the sheath 504 removed, the paddle lead 107 can be coupled to a control module (*e.g.*, 102 of Figure 1) and implanted using well-known techniques, for example, using one or more using tunneling straws placed in passageways underneath patient skin with bores that are sized large enough to receive the paddle lead 107. In at least some embodiments, the paddle lead 107 can be coupled to a connector of a control module, as shown in Figure 2A. In other embodiments, the paddle lead 107 can be coupled to one or more other devices, including an adaptor, a lead extension (see *e.g.*, Figure 2B), an operating room cable, or the like or combinations thereof.

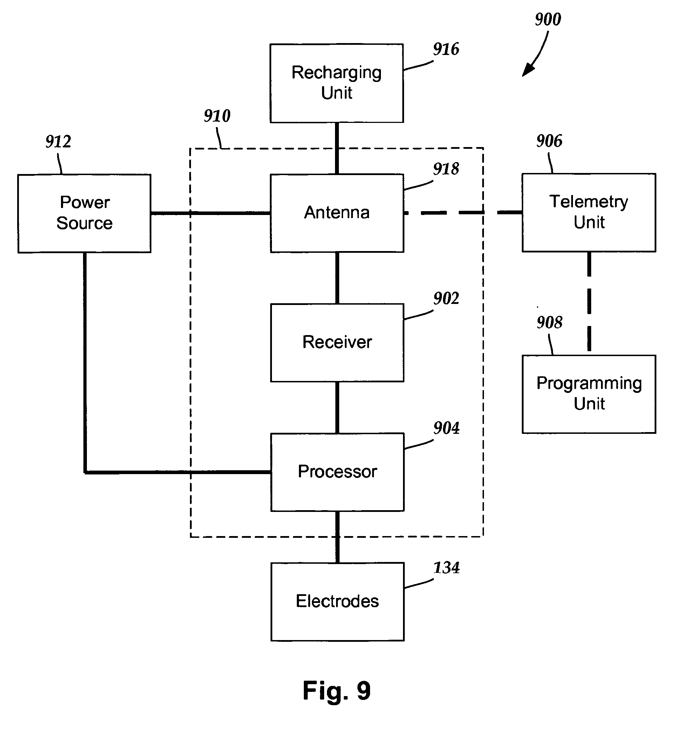

Figure 9 is a schematic overview of one embodiment of components of an electrical stimulation system 900 including an electronic subassembly 910 disposed within a control module. It will be understood that the electrical stimulation system can include more, fewer, or different components and can have a variety of different configurations including those configurations disclosed in the stimulator references cited herein.

Some of the components (for example, power source 912, antenna 918, receiver 902, and processor 904) of the electrical stimulation system can be positioned on one or more circuit boards or similar carriers within a sealed housing of an implantable pulse generator, if desired. Any power source 912 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent. No. 7,437,193, incorporated herein by reference.

As another alternative, power can be supplied by an external power source through inductive coupling via the optional antenna 918 or a secondary antenna. The external power source can be in a device that is mounted on the skin of the user or in a unit that is provided near the user on a permanent or periodic basis.

If the power source 912 is a rechargeable battery, the battery may be recharged using the optional antenna 918, if desired. Power can be provided to the battery for recharging by inductively coupling the battery through the antenna to a recharging unit 916 external to the user, Examples of such arrangements can be found in the references identified above.

In one embodiment, electrical current is emitted by the electrodes 134 on the paddle or lead body to stimulate nerve fibers, muscle fibers, or other body tissues near the electrical stimulation system. A processor 904 is generally included to control the timing and electrical characteristics of the electrical stimulation system. For example, the processor 904 can, if desired, control one or more of the timing, frequency, strength, duration, and waveform of the pulses. In addition, the processor 904 can select which electrodes can be used to provide stimulation, if desired. In some embodiments, the processor 904 may select which electrode(s) are cathodes and which electrode(s) are anodes. In some embodiments, the processor 904 may be used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 908 that, for example, allows modification of pulse characteristics. In the illustrated embodiment, the processor 904 is coupled to a receiver 902 which, in turn, is coupled to the optional antenna 918. This allows the processor 904 to receive instructions from an external source to, for example, direct the pulse characteristics and the selection of electrodes, if desired.

In one embodiment, the antenna 918 is capable of receiving signals (*e.g.*, RF signals) from an external telemetry unit 906 which is programmed by a programming unit 908. The programming unit 908 can be external to, or part of, the telemetry unit 906. The telemetry unit 906 can be a device that is worn on the skin of the user or can be carried by the user and can have a form similar to a pager, cellular phone, or remote control, if desired. As another alternative, the telemetry unit 906 may not be worn or carried by the user but may only be available at a home station or at a clinician's office. The programming unit 908 can be any unit that can provide information to the telemetry unit 906 for transmission to the electrical stimulation system 900. The programming unit 908 can be part of the telemetry unit 906 or can provide signals or information to the telemetry unit 906 via a wireless or wired connection. One example of a suitable programming unit is a computer operated by the user or clinician to send signals to the telemetry unit 906.

The signals sent to the processor 904 via the antenna 918 and receiver 902 can be used to modify or otherwise direct the operation of the electrical stimulation system. For example, the signals may be used to modify the pulses of the electrical stimulation system such as modifying one or more of pulse duration, pulse frequency, pulse waveform, and pulse strength. The signals may also direct the electrical stimulation system 900 to cease operation, to start operation, to start charging the battery, or to stop charging the battery. In other embodiments, the stimulation system does not include an antenna 918 or receiver 902 and the processor 904 operates as programmed.

Optionally, the electrical stimulation system 900 may include a transmitter (not shown) coupled to the processor 904 and the antenna 918 for transmitting signals back to the telemetry unit 906 or another unit capable of receiving the signals. For example, the electrical stimulation system 900 may transmit signals indicating whether the electrical stimulation system 900 is operating properly or not or indicating when the battery needs to be charged or the level of charge remaining in the battery. The processor 904 may also be capable of transmitting information about the pulse characteristics so that a user or clinician can determine or verify the characteristics.

## Claims

1. An insertion kit for percutaneously implanting an electrical stimulating paddle lead (107) into a patient, the insertion kit comprising:
a paddle lead introducer (502) configured and arranged for facilitating percutaneous implantation of a paddle lead (107) into the patient, the paddle lead introducer (502) comprising
a sheath (504) insertable into the patient, the sheath (504) having a first end (510), an opposing second end (512), and a longitudinal length (514), wherein the sheath (504) is configured and arranged to receive a paddle lead (107) during implantation of the paddle lead (107) into the patient, wherein the sheath (504) is configured and arranged to divide into at least two parts for removal of the sheath (504) from the paddle lead (107) upon implantation of the paddle lead (107) into the patient, and
a dilator (506) insertable into the sheath (504), the dilator (506) having a first end (520), an opposing second end (522), and a longitudinal length (524), the first end (520) of the dilator (506) defining an aperture (530) at a tip of the first end (520), wherein the first end (520) of the dilator (506) has a transverse circumference that increases from the tip towards the second end (522); and
an epidural needle (540) having a first end (550), an opposing second end (552), and a longitudinal length, wherein the epidural needle (540) is insertable into the dilator (506) such that the first end (550) of the epidural needle extends outwardly from the aperture at the tip of the first end (520) of the dilator (506).

2. The insertion kit of claim 1, wherein the sheath (504) has an oval-shaped transverse cross-section.

3. The insertion kit of claim 1, wherein the dilator (506) has an oval-shaped transverse cross-section.

4. The insertion kit of claim 1, wherein the first end (510) of the sheath (504) is beveled.

5. The insertion kit of claim 1, wherein the first end (510) of the sheath (504) has a sharp surface.

6. The insertion kit of claim 1, wherein the sheath (504) comprises at least two pull-apart tabs (518a, 518b) disposed at opposite sides of the second end (512) of the sheath (504).

7. The insertion kit of claim 6, wherein the sheath (504) further comprises at least one scored or perforated line (516) extending along at least a portion of a longitudinal length of the sheath (504) from between the at least two pull-apart tabs (518a, 518b), the at least one scored or perforated line (516) configured and arranged for separating when the at least two pull-apart tabs (518a, 518b) are pulled apart from one another.

8. The insertion kit of claim 1, wherein the epidural needle (540) is a 14-gauge epidural needle.

9. The insertion kit of claim 1, wherein the epidural needle (540) comprises a luer hub (556) disposed at the second end (552) of the epidural needle (540).

10. The insertion kit of claim 1, further comprising a lead blank (570) insertable into the epidural needle (540).

11. The insertion kit of claim 1, further comprising a paddle lead (107), the paddle lead (502) comprising
a paddle body (104) having a first major surface, an opposing second major surface, and a longitudinal length;
an aperture defined in the paddle body (104), wherein the aperture extends along the entire longitudinal length of the paddle body (104), the aperture configured and arranged to receive a lead blank (570);
at least one lead body (106), each of the at least one lead bodies (106) having a proximal end and a distal end, the distal end of the at least one lead body (106) being coupled to the paddle body (104);
a plurality of electrodes (134) disposed on the first major surface of the paddle body (104);
a plurality of terminals disposed at the proximal ends of each of the at least one lead bodies (106); and
a plurality of conductive wires coupling the plurality of electrodes (134) electrically to the plurality of terminals.

12. The insertion kit of claim 11, wherein at least one of the at least one lead bodies (106) defines a lumen configured and arranged to receive a stylet.

13. An electrical stimulating system (100) comprising:
the insertion kit of claim 11;
a control module (102) configured and arranged to electrically couple to the proximal ends of the at least one lead bodies (106) of the paddle lead (107), the control module (102) comprising
a housing (114), and
an electronic subassembly (110) disposed in the housing (114); and
a connector (144) for receiving the paddle lead (107), the connector (144) comprising a connector housing (214) defining at least one port (204) for receiving the proximal ends of the at least one lead bodies (106) of the paddle lead (107), and
a plurality of connector contacts (216) disposed in the connector housing (214), the connector contacts (216) configured and arranged to couple to the plurality of terminals disposed at the proximal ends of the at least one lead bodies (106).

14. The insertion kit of claim 11, wherein the plurality of electrodes (134) are configured into at least two columns of electrodes (134) each extending along the longitudinal length of the paddle body (104).

15. The insertion kit of claim 11, wherein the plurality of electrodes (134) comprises sixteen electrodes.

## Patentansprüche

1. Einführungssatz zum perkutanen Implantieren einer elektrischen Stimulationspaddelleitung (107) in einen Patienten, wobei der Einführungssatz aufweist:
einen Paddelleitungs-Introducer (502), der zur Erleichterung der perkutanen Implantation einer Paddelleitung (107) in den Patienten konfiguriert und eingerichtet ist, wobei der Paddelleitungs-Introducer (502) aufweist
eine Hülle (504), die in den Patienten einführbar ist, wobei die Hülle (504) ein erstes Ende (510), ein gegenüberliegendes zweites Ende (512) und eine longitudinale Länge (514) aufweist, wobei die Hülle (504) konfiguriert und eingerichtet ist, eine Paddelleitung (107) während der Implantation der Paddelleitung (107) in den Patienten aufzunehmen, wobei die Hülle (504) konfiguriert und eingerichtet ist, sich zur Entfernung der Hülle (504) von der Paddelleitung (107) bei der Implantation der Paddelleitung (107) in den Patienten in mindestens zwei Teile aufzuteilen, und
einen Dilator (506), der in die Hülle (504) einführbar ist, wobei der Dilator (506) ein erstes Ende (520), ein gegenüberliegendes zweites Ende (522) und eine longitudinale Länge (524) aufweist, wobei das erste Ende (520) des Dilators (506) eine Öffnung (530) an einer Spitze des ersten Endes (520) definiert, wobei das erste Ende (520) des Dilators (506) eine transversale Ausdehnung aufweist, die von der Spitze zum zweiten Ende (522) zunimmt; und
eine epidurale Nadel (540), die ein erstes Ende (550), ein gegenüberliegendes zweites Ende (552) und eine longitudinale Länge aufweist, wobei die epidurale Nadel (540) in den Dilator (506) einführbar ist, so dass sich das erste Ende (550) der epiduralen Nadel aus der Öffnung an der Spitze des ersten Endes (520) des Dilators (506) nach außen erstreckt.

2. Einführungssatz nach Anspruch 1, wobei die Hülle (504) einen ovalen transversalen Querschnitt aufweist.

3. Einführungssatz nach Anspruch 1, wobei der Dilator (506) einen ovalen transversalen Querschnitt aufweist.

4. Einführungssatz nach Anspruch 1, wobei das erste Ende (510) der Hülle (504) abgeschrägt ist.

5. Einführungssatz nach Anspruch 1, wobei das erste Ende (510) der Hülle (504) eine scharfe Oberfläche aufweist.

6. Einführungssatz nach Anspruch 1, wobei die Hülle (504) mindestens zwei Auftrennungsansätze (518a, 518b) aufweist, die auf gegenüberliegenden Seiten des zweiten Endes (512) der Hülle (504) angeordnet sind.

7. Einführungssatz nach Anspruch 6, wobei die Hülle (504) ferner mindestens eine gepunktete oder perforierte Linie (516) aufweist, die sich längs mindestens eines Abschnitts einer longitudinalen Länge der Hülle (504) von zwischen den mindestens zwei Auftrennungsansätzen (518a, 518b) erstreckt, wobei die mindestens eine gepunktete oder perforierte Linie (516) zur Trennung konfiguriert und eingerichtet ist, wenn die mindestens zwei Auftrennungsansätze (518a, 518b) voneinander weg auseinandergezogen werden.

8. Einführungssatz nach Anspruch 1, wobei die epidurale Nadel (540) eine epidurale Nadel mit 14 Gauge ist.

9. Einführungssatz nach Anspruch 1, wobei die epidurale Nadel (540) einen Luer-Anschluss (556) aufweist, der am zweiten Ende (552) der epiduralen Nadel (540) angeordnet ist.

10. Einführungssatz nach Anspruch 1, der ferner ein Leitungsblindstück (570) aufweist, das in die epidurale Nadel (540) einführbar ist.

11. Einführungssatz nach Anspruch 1, der ferner eine Paddelleitung (107) aufweist, wobei die Paddelleitung aufweist
einen Paddelkörper (104), der eine erste Hauptfläche, eine gegenüberliegende zweite Hauptfläche und eine longitudinale Länge aufweist;
eine Öffnung, die im Paddelkörper (104) definiert ist, wobei sich die Öffnung längs der gesamten longitudinalen Länge des Paddelkörpers (104) erstreckt, und die Öffnung konfiguriert und eingerichtet ist, ein Leitungsblindstück (570) aufzunehmen;
mindestens einen Leitungskörper (106), wobei jeder des mindestens einen Leitungskörpers (106) ein proximales Ende und ein distales Ende aufweist, wobei das distale Ende des mindestens einen Leitungskörpers (106) mit dem Paddelkörper (104) verbunden ist; mehrere Elektroden (134), die an der ersten Hauptfläche des Paddelkörpers (104) angeordnet sind;
mehrere Anschlüsse, die an den proximalen Enden jedes des mindestens einen Leitungskörpers (106) angeordnet sind; und
mehrere leitfähige Drähte, die die mehreren Elektroden (134) elektrisch mit den mehreren Anschlüssen verbinden.

12. Einführungssatz nach Anspruch 11, wobei mindestens einer des mindestens einen Leitungskörpers (106) ein Lumen definiert, das konfiguriert und eingerichtet ist, ein Stilett aufzunehmen.

13. Elektrisches Stimulationssystem (100), das aufweist:
den Einführungssatz nach Anspruch 11;
ein Steuermodul (102), das konfiguriert und eingerichtet ist, sich elektrisch mit den proximalen Enden des mindestens einen Leitungskörpers (106) der Paddelleitung (107) zu verbinden, wobei das Steuermodul (102) aufweist
ein Gehäuse (114), und
eine elektronische Baugruppe (110), die im Gehäuse (114) angeordnet ist; und
eine Anschlussbuchse (144) zur Aufnahme der Paddelleitung (107), wobei die Anschlussbuchse (144) ein Anschlussbuchsengehäuse (214) aufweist, das mindestens eine Öffnung (204) zur Aufnahme der proximalen Enden des mindestens einen Leitungskörpers (106) der Paddelleitung (107) definiert, und
mehrere Anschlussbuchsenkontakte (216), die im Anschlussbuchsengehäuse (214) angeordnet sind, wobei die Anschlussbuchsenkontakte (216) konfiguriert und eingerichtet sind, sich mit den mehreren Anschlüssen zu verbinden, die an den proximalen Enden des mindestens einen Leitungskörpers (106) angeordnet sind.

14. Einführungssatz nach Anspruch 11, wobei die mehreren Elektroden (134) in mindestens zwei Spalten von Elektroden (134) konfiguriert sind, die sich jeweils längs der longitudinalen Länge des Paddelkörpers (104) erstrecken.

15. Einführungssatz nach Anspruch 11, wobei die mehreren Elektroden (134) sechzehn Elektroden umfassen.

## Revendications

1. Kit d'insertion pour l'implantation percutanée sur un patient d'une dérivation à palette de stimulation électrique (107), ledit kit d'insertion comprenant :
un introducteur (502) de dérivation à palette conçu et agencé pour faciliter l'implantation percutanée d'une dérivation à palette (107) sur le patient, ledit introducteur (502) de dérivation à palette comprenant
une gaine (504) insérable sur le patient, ladite gaine (504) présentant une première extrémité (510), une deuxième extrémité (512) opposée et une longueur longitudinale (514), ladite gaine (504) étant conçue et agencée pour recevoir une dérivation à palette (107) pendant l'implantation de la dérivation à palette (107) sur le patient, ladite gaine (504) étant conçue et agencée pour se diviser en au moins deux parties pour le retrait de la gaine (504) de la dérivation à palette (107) lors de l'implantation de la dérivation à palette (107) sur le patient, et
un dilatateur (506) insérable dans la gaine (504), ledit dilatateur (506) présentant une première extrémité (520), une deuxième extrémité (522) opposée et une longueur longitudinale (524), la première extrémité (520) du dilatateur (506) définissant une ouverture (530) à un sommet de la première extrémité (520), la première extrémité (520) du dilatateur (506) ayant une circonférence transversale qui augmente du sommet vers la deuxième extrémité (522) ; et
une aiguille péridurale (540) présentant une première extrémité (550), une deuxième extrémité (552) opposée, et une longueur longitudinale, ladite aiguille péridurale (540) étant insérable dans le dilatateur (506) de telle manière que la première extrémité (550) de l'aiguille péridurale s'étend à l'extérieur de l'ouverture au sommet de la première extrémité (520) du dilatateur (506).

2. Kit d'insertion selon la revendication 1, où la gaine (504) a une section transversale de forme ovale.

3. Kit d'insertion selon la revendication 1, où le dilatateur (506) a une section transversale de forme ovale.

4. Kit d'insertion selon la revendication 1, où la première extrémité (510) de la gaine (504) est biseautée.

5. Kit d'insertion selon la revendication 1, où la première extrémité (510) de la gaine (504) présente une surface tranchante.

6. Kit d'insertion selon la revendication 1, où la gaine (504) comporte au moins deux pattes de traction (518a, 518b) disposées sur des côtés opposés de la deuxième extrémité (512) de la gaine (504).

7. Kit d'insertion selon la revendication 6, où la gaine (504) comporte en outre au moins une ligne entaillée ou perforée (516) qui s'étend sur au moins une partie d'une longueur longitudinale de la gaine (504) depuis un point entre les au moins deux pattes de traction (518a, 518b), la ou les lignes entaillées ou perforées (516) étant conçues et agencées pour se déchirer quand les au moins deux pattes de traction (518a, 518b) sont séparées l'une de l'autre par traction.

8. Kit d'insertion selon la revendication 1, où l'aiguille péridurale (540) est une aiguille péridurale de calibre 14.

9. Kit d'insertion selon la revendication 1, où l'aiguille péridurale (540) comporte un raccord Luer verrouillable (556) disposé à la deuxième extrémité (552) de l'aiguille péridurale (540).

10. Kit d'insertion selon la revendication 1, comprenant en outre une âme de dérivation (570) insérable dans l'aiguille péridurale (540).

11. Kit d'insertion selon la revendication 1, comprenant en outre une dérivation à palette (107), ladite dérivation à palette comprenant
un corps de palette (104) avec une première surface principale, une deuxième surface principale opposée, et une longueur longitudinale ;
une ouverture définie dans le corps de palette (104), ladite ouverture sur toute la longueur longitudinale du corps de palette (104), ladite ouverture étant conçue et agencée pour recevoir une âme de dérivation (570) ;
au moins un corps de dérivation (106), chaque corps de dérivation (106) ayant une extrémité proximale et une extrémité distale, l'extrémité distale du ou des corps de dérivation (106) étant reliée au corps de palette (104) ;
une pluralité d'électrodes (134) disposées sur la première surface principale du corps de palette (104) ;
une pluralité de bornes disposées à l'extrémité proximale de chaque corps de dérivation (106) ; et
une pluralité de fils conducteurs reliant électriquement la pluralité d'électrodes (134) à la pluralité de bornes.

12. Kit d'insertion selon la revendication 11, où au moins un corps de dérivation (106) définit une lumière conçue et agencée pour recevoir un stylet.

13. Système de stimulation électrique (100), comprenant :
le kit d'insertion selon la revendication 11 ;
un module de commande (102) conçu et agencé pour être raccordé électriquement à les extrémités proximales du ou des corps (106) de la dérivation à palette (107), ledit module de commande (102) comprenant
un boîtier (114), et
un sous-ensemble électronique (110) disposé dans le boîtier (114) ; et
un connecteur (144) pour la réception de la dérivation à palette (107), ledit connecteur (144) comprenant un boîtier de connecteur (214) définissant au moins un port (204) pour la réception des extrémités proximales du ou des corps (106) de la dérivation à palette (107), et
une pluralité de contacts de connecteur (216) disposés dans le boîtier de connecteur (214), lesdits contacts de connecteur (216) étant conçus et agencés pour être connectés à la pluralité de bornes disposées à l'extrémité proximale du ou des corps de dérivation (106).

14. Kit d'insertion selon la revendication 11, où la pluralité d'électrodes (134) est agencée en au moins deux colonnes d'électrodes (134) s'étendant chacune sur la longueur longitudinale du corps de palette (104).

15. Kit d'insertion selon la revendication 11, où la pluralité d'électrodes (134) comprend seize électrodes.
